# EUROPEAN PATENT APPLICATION

(11) **EP 1 331 222 A1**
(43) Date of publication of application: **30.07.2003**
(21) Application number: 02290184.7
(22) Date of filing: 28.01.2002
(51) Int. Cl.: C07D 207/48, C07D 209/48, C07D 207/34, C07D 231/44, C07C 303/36, C07C 311/02, C07C 303/28, C07C 309/04

(54) **Environment friendly reagents and process for halogenoalkylsulfinylation of organic compounds**

(71) Applicant: VIRBAC S.A., F-06516 Carros Cedex (FR)
(72) Inventor: Bertrand, Guy, 92506 Riverside, California (US); Romanenko, Vadim D., The Greacian Apartments, Riverside, CA 92507 (US); Raynier, Bernard, 06200 Nice (FR); Derrieu, Guy, 06800 Cagnes sur Mer (FR)
(74) Representative: Corizzi, Valérie

(57) **Abstract**

The invention concerns compounds corresponding to formula (I) : 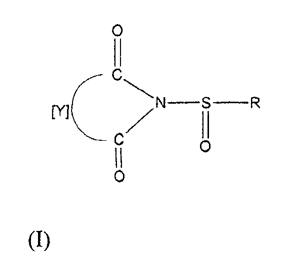 wherein:
- Y represents a group chosen from : a C₁-C₂₀, linear, branched or cyclic alkane di-yl, or alkene di-yl, or alkyne di-yl radical, wherein said alkane, alkene or alkyne chain can be substituted by one or more groups chosen from : a phenyl ring or a halogen atom, and said alkane, alkene or alkyne chain can comprise a sulfur bridge or an oxygen bridge ;
- R represents an alkyl group, linear or branched, comprising one to four carbon atoms and substituted by one or more, identical or different, halogen atoms ;
   a process for their preparation and their use as halogenoalkylsulfinylating agents.

## Description

This invention relates to new environment friendly reagents and process for electrophilic halogenoalkylsulfinylation, and especially to reagents and process for trifluoromethylsulfinylation, of organic compounds.

The introduction of CF₃S(O)-group into organic molecules (trifluoromethylsulfinylation reaction) has become an important method in organic chemistry since the biological importance of many products bearing this substituent has been discovered. As one of the most lipophilic structural moiety known at the moment, the CF₃S(O) group is currently widely involved in the design of biologically-active compounds, both in pharmaceutical and agrochemical fields. For example, the trifluoromethylsulfinyl group is present in the structure of the second-generation N-arylpyrazole insecticides which are nanomolar inhibitors of the gamma-aminobutyric acid gated chloride channel such as potent insecticide FIPRONIL (EP 0 295 117 A1; US Patent 5,232,940) and its several analogues (IP WO 98/28279; EP 0 201 852 B1; EP 0 216 102 A1; DE 196 50 197 A1; DE 198 53 560 A1). In fact, the substitution of a proton by CF₃S(O) increases lipid solubility of a given molecule, and this increases the rate of transport of a biologically active molecule across lipid membranes. As a consequence, the introduction of CF₃S(O) group into organic molecules often increases or changes their physiological activity.

The synthesis of trifluoromethylsulfinyl derivatives is generally not trivial task : Existing routes to trifluoromethylsulfinyl-functionalized compounds are not numerous and usually employ multistep procedures and highly toxic non-commercial reagents. Purification of the products is difficult at best, and yields vary widely, depending on the nature of the substrate.

The more often practised preparative method for the synthesis of sulfinyl compounds is based on nucleophilic substitution at tricoordinate sulfur(IV) atom as is represented in equation 1 (Eq.1):

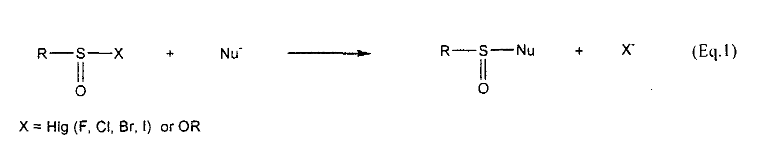

Application of this approach for the preparation of halogenoalkylsulfinyl derivatives and especially trifluoromethylsulfinyl derivatives (Eq.1, R = CF₃) has been well documented in the literature (Shreeve and co-workers, J. Am. Chem. Soc., 1968, vol. 90, p. 5403-5408; Inorg. Chem., 1971, vol. 10, p. 358-362; ibid, 1977, vol. 16, p. 1039-1042; ibid, 1985, vol. 24, p.2126-2129 ). It has been found that trifluoromethylsulfinyl halides (X = F, Cl) and some trifluoromethylsulfinyl esters (X = OR) react with different organic nucleophiles including amines, alcohols and N-heterocyclic compounds to form the corresponding trifluoromethylsulfinyl derivatives. However, specific difficulties and limitations are associated with this method due to numerous drawbacks of the above mentioned compounds as sulfinylating reagents :
(i) sulfinyl halides with general formula CF₃S(O)Hlg, wherein H1g represents a halogen atom, have low stability : trifluoromethylsulfinyl fluoride is a light sensitive compound, the corresponding chloride tends to decompose to the respective sulfonyl and sulfenyl halides (CF₃SO₂Cl and CF₃SCl) at room temperature and can be stored only for a short time in a freezer ; bromide and iodide are unstable and have not been isolated in pure state ;
(ii) trifluoromethylsulfinyl halides are highly volatile and physiologically dangerous: CF₃S(O)F is a gaz having a boiling point of approximately - 9 °C ; CF₃S(O)Cl has a boiling point around 41 °C ;
(iii) because of the low thermal stability of CF₃S(O)Cl, the utility of the compound for the introduction of CF₃S(O) moiety into organic molecules is limited to low-temperature conditions ;
(iv) trifluoromethylsulfinyl chloride is highly air-sensitive ; in a polar media and in the presence of moisture or organic bases, usually used as HCl-binding agents, the rate of decomposition of the compound grows rapidly;
(v) the isolation and purification of products is difficult as the solubilities of trifluoromethylsulfinyl-derivatives and hydrochloride salts of organic bases are similar;
(vi) although trifluoromethylsulfinyl esters display higher stability and are less volatile in comparison with the corresponding halides, they are not reactive enough toward most organic substrates.

In order to avoid the decomposition of trifluoromethylsulfinyl chloride in carrying out the process, a special technique was developed such as mixing an organic base and the substrate prior to the addition of the reagent (Shreeve and co-workers, Inorg. Chem., 1985, vol. 24, p. 2126-2129), generation of CF₃S(O)Cl *in situ* from the corresponding sulfinate salts and phosgene or thionyl chloride just before subsequent reactions, as well as the use of low basic HCl-binding agent, for example, anhydrous dimethylamine para-toluenesulfonate (EP 0668 269 A1). In addition, methods for generating reactive species behaving like trifluoromethylsulfinating reagents *in situ*, by treatment of CF₃SO₂Na with phosphoryl chloride or trifluoromethanesulfonic acid have recently been proposed (Langlois and co-workers, Tetrahedron, 1999, vol.35, p.7243-7250 ; Wakselman and co-workers, Synlett, 2001, No 4, p. 550-552). Unfortunately, these developments do not get rid of the drawbacks peculiar to CF₃S(O)Cl. Moreover, difficulties can arise from the fact that these highly reactive species are not compatible with many organic functional groups.

Anionic route to trifluoromethylsulfinyl compounds has recently been proposed (Yagupolskii and co-workers, J. Fluor. Chem., 1995, vol.70, p. 255-257) and is illustrated in Equation 2 (Eq. 2). However, although the trifluoromethyl anion is easily generated from readily available precursors, this method has very limited applicability since functionalised sulfinyl chlorides are largely inaccessible.

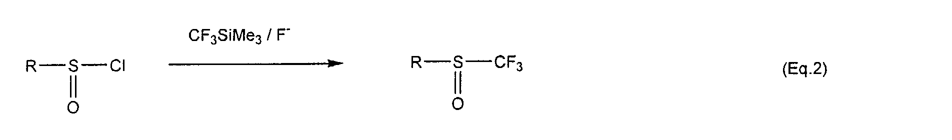

An alternative method for the preparation of trifluoromethylsulfinyl derivatives utilizes the oxidation of the corresponding sulfides with organic peracids (Eq. 3):

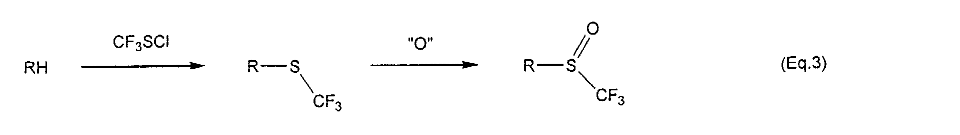

This type of reactions has been applied extensively to the preparation of many alkyl- and aryl-sulfoxides (Umemoto and Ishihara, J. Am. Chem. Soc., 1993, vol. 115, p. 2156-216; Shreeve and co-workers, J. Org. Chem., 1998, vol. 63, p. 2656-2660). However, in contrast to easily available non-fluorinated organic substrates, trifluoromethyl-substituted sulfide is difficult to prepare. Synthesis of the latter requires the use of highly toxic non commercial trifluoromethylsulfenyl chloride (CF₃SCl). In addition, the oxidation reaction leads not only to the desired sulfoxides, but also the corresponding sulfones and sulfides and, in a number of instances, the separation of the trifluoromethylsulfoxide from the reaction mixture has been difficult.

Obviously, the development of new, efficient, environment friendly reagents and practical method for high-yielding trifluoromethylsulfinylation of organic compounds is of considerable economic and scientific interest.

A comparison of different synthetic approaches to trifluoromethylsulfinyl derivatives shows that a nucleophilic substitution at three-coordinate S(IV) atom as illustrated in Equation 1 (Eq. 1) has significant advantages with regards to versatility and scope of application. Therefore the search for a new synthetic auxiliary that can conveniently play the role of halogenoalkylsulfinyl group transfer, and especially CF₃S(O) group transfer, as well as a new method for electrophilic halogenoalkyl sulfinylation and especially trifluoromethylsulfinylation of organic compounds is the essence of the present invention.

As was mentioned above, among the three classes of trifluoromethylsulfinyl derivatives - halides, esters and amides - only trifluoromethylsulfinyl halides are efficient electrophilic sulfinating reagents. Rhone-Poulenc patent (EP 0 668 269 A1) claims that compounds with general formula RS(O)NR¹R², wherein R¹ and R² represent alkyl or haloalkyl groups containing from 1 to 4 carbon atoms, and R represents a halogenoalkyl group, possess halogenoalkylsulfinilating activity. However, the latter are not reactive toward most of organic nucleophiles due to the essential contribution of resonance form B in which the positive nature of the S(IV) atom is not retained.

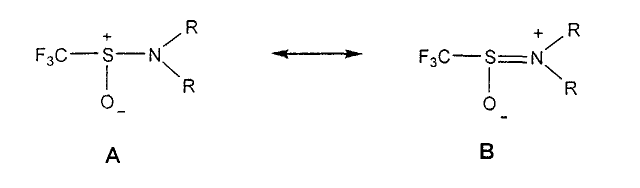

It has now been found after extensive research that the compounds of the general formula (I) depicted hereinafter, in which two carbonyl groups are linked to the nitrogen atom are powerful halogenoalkylsulfinyl- transfer reagents, and especially CF₃S(O)-transfer reagents. Moreover, they have significant advantages with respect to halogenoalkylsulfinyl halides, and especially trifluoromethylsulfinyl halides as is made clear in the following description.

The first object of the present invention is the compounds corresponding to formula (I) wherein : 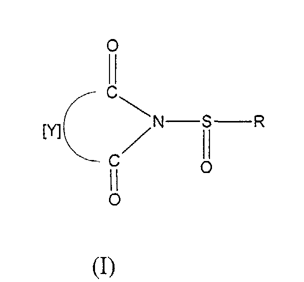
- Y represents a group chosen from : a C₁-C₂₀, linear, branched or cyclic alkane di-yl, or alkene di-yl, or alkyne di-yl radical, wherein said alkane, alkene or alkyne chain can be substituted by one or more groups chosen from : a phenyl ring or a halogen atom, like for example F, Cl, Br, I, wherein said alkane, alkene or alkyne chain can comprise a sulfur bridge or an oxygen bridge ;
- R represents an alkyl group, linear or branched comprising one to four carbon atoms and substituted by one or more, identical or different, halogen atoms.

According to a variant of the invention, said alkane, alkene or alkyne chain Y can bear two or more di-radicals according to formula (II) : 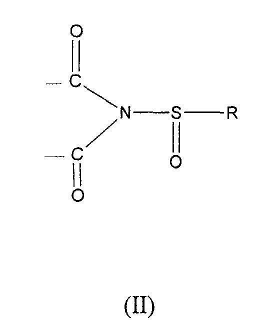 wherein R has the same meaning as above. The resultant molecule has the property of being able to transfer two or more halogenoalkylsulfinyl groups to the substrate. Said molecule (I) will then be introduced in half or less molar proportion with regard to the substrate to be treated.

Preferred compounds of general formula (I) are those in which Y is chosen so that the radical of formula (III) : 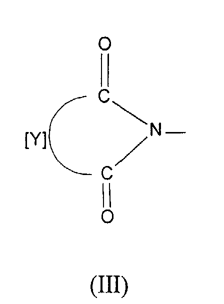 is corresponding to different 5- or 6-membered cyclic dicarboximide derivatives such as succinimidyl (A), tetrahydrophthalimidyl (B), endo-5-norbornene-3,3-dicarboximidyl (C), 2,4-thiazolidenedion-3-yl (D), glutarimidyl (E), maleinimidyl (F), phtalimidyl (G), tetrachlorophthalimidyl (H), 1,8-naphthalimidyl (I), pyrromellitic diimidyl (J):

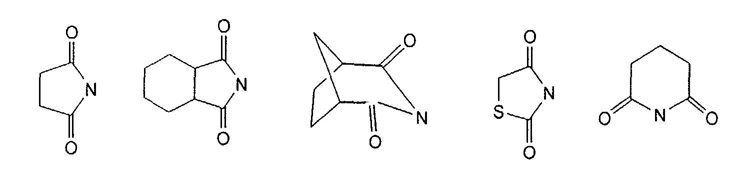

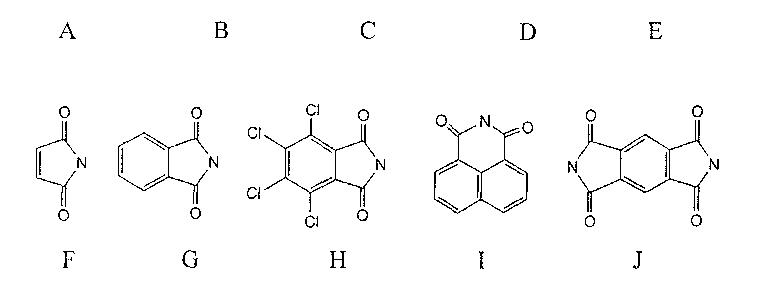

Compounds of general formula (I) wherein the heterocyclic moiety (III) represents a group chosen from: succinimidyl (A), phthalimidyl (G) and tetrachlorophthalimidyl (H) are especially preferred.

Preferentially R represents trifluoromethyl.

None of the foregoing publications describes or even suggests that compounds of general formula (I) are stable and possess or would be expected to possess the powerful halogenoalkylsulfinylating, and particularly, trifluoromethylsulfinylating activity, toward a wide range of nucleophilic substrates. Moreover, it has been reported that non-halogenated analogues of the compounds (I) - N-(phenylsulfinyl)- and N-(benzylsulfinyl)-succinimides - were not isolable in pure state and could only be stored in solution at 0 °C for a short period of time (J. Org. Chem., 1980, vol. 45, p. 5336).

According to the present invention, the compounds of general formula (I) can be easily prepared from the corresponding N-metal substituted cyclic imides and halogenoalkylsulfinylchloride as represented in Reaction 1 : 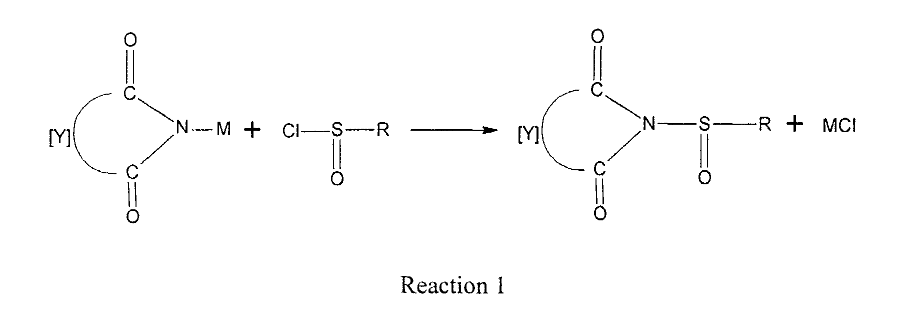 wherein M represents a metal atom, like, for example a lithium, sodium, potassium, magnesium, silver, copper atom, and Y and R have the same meaning as above. The reaction is generally made in a halogenocarbonated solvent. Thus, N-lithium phthalimide readily reacts with CF₃S(O)Cl in chlorobenzene at room temperature, giving the stable N-trifluoromethylsulfinylphtalimide in 76 % yield. Similarly, N-lithium and N-silver substituted succinimides reacts with CF₃S(O)Cl to form the corresponding N-trifluoromethylsuccinimide in high yield (Example 1). Both compounds are white solids and stable on storage. The N-metal substituted cyclic imides that are especially interesting for the synthesis of the compounds with general formula (I) are commercial products (Aldrich Co.) or can be prepared by methods described in the literature (see, for example, K. Soal and co-workers, Bull. Chem. Soc. Jpn., 1982, vol. 55, p. 1671-1672).

The electron-withdrawing nature of the imide cycle in the compounds according to formula (I) is responsible for their tendency to act as effective CF₃S(O)⁺ transfer reagents. Unlike trifluoromethylsulfinyl amides of formula CF₃S(O)NR¹R², wherein R¹ and R² represent alkyl or aryl group, the contribution from resonance form (B) in compounds (I) is weak due to delocalisation of the nitrogen lone pair with participation of the neighbouring carbonyl groups. Moreover, the imidic substituent exerts activating influence on CF₃S(O) group in electrophilic substitution reactions as can be seen from strong deshielding the 19F NMR signal in N-trifluoromethylsulfinylsuccinimide as compared to those of the corresponding amides, esters and triflinate salts

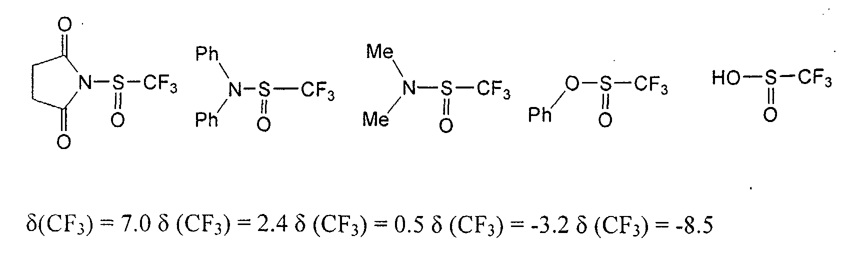

The present invention also discloses a novel and convenient method for the introduction of halogenoalkylsulfinyl groups, and especially CF₃S(O) group into organic compounds. We have found that reaction of a compound of general formula (I) with organic substrates noted NuH containing N-H, O-H and activated C-H bonds may be used as a general method for the synthesis of trifluoromethylsulfinylated derivatives (Examples 2-4). The process can be represented by the following equation:

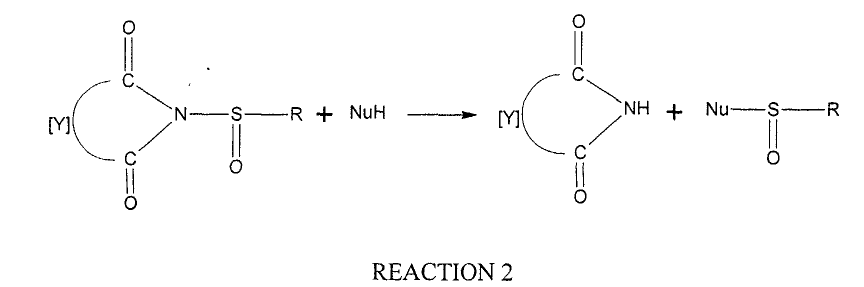

For carrying out the process, 1.0 to 1.2 moles of a compound of the general formula (I) are employed per mole of nucleophilic substrate. The nucleophilic substrate is preferentially chosen from : alcohols, phenols, amines and heterocyclic compounds. Among heterocyclic compounds, favorites are : pyrroles, pyrazoles, imidazoles, oxazoles, isoxazoles, isothiazoles, thiazoles, triazoles, said heterocycles being possibly substituted by one or more atom or groups chosen from : a halogen atom, -NH₂, a C₁-C₁₀ mono- or di-alkylamine, a nitrile, one or more C₁-C₁₀ alkyl or C₁-C₁₀ halogenoalkyl group, an aryle group, said aryle group being possibly substituted by one or more halogen atom, one or more C₁-C₁₀ alkyl or C₁-C₁₀ halogenoalkyl group. Suitable solvents are chlorinated hydrocarbons, such as dichloromethane, chloroform or 1,2-dichloroethane, or aromatic, optionally halogenated, hydrocarbons such as, for example, toluene and chlorobenzene. In a preferred procedure, the solution of compound (I) in organic solvent is added at temperature between 0 °C and 90 °C to a nucleophilic substrate in the absence or in the presence of an organic base such as triethylamine or pyridine. After stirring for 1-3 h the reaction product is worked up and isolated by generally customary methods. The yields are good to excellent and the workup is easy. The succinimide and related cyclic imides forming as by-product are sparingly soluble in organic solvents and can be collected by filtration or separated from the desired product by washing with water. The resulting crude trifluoromethylsulfinyl derivatives are almost pure and show NMR spectra very close to those of purified compounds. The cyclic imide can be conveniently regenerated and reconverted into compound (I).

The use of a compound of general formula (I) as defined according to the present invention, is of particularly remarquable advantage in the reaction with low-nucleophilic organic substrates since the stability of (I) toward organic bases, such as triethylamine, as well as their thermal stability, permits to avoid the employment of special costly and time-consuming manipulations of large excess of highly air-sensitive p-toluenesulfonate salts Thus, 5-amino-3-methyl-1-phenylpyrazol smoothly reacts with N-trifluoromethylsulfinylsuccinimide at 80 °C to form the corresponding 4-trifluoromethylsulfinylated derivative in almost quantitative yield (Example 5). 5-Amino-3-cyano-4-trifluoromethylsulfinyl-1-(2,6-dichloro-4-trifluoromethyl-phenyl)pyrazole could not be obtained in similar conditions. However, the corresponding 4-unsubstituted pyrazole has been easily trifluoromethylsulfinylated in the presence of 1 equivalent of triethylamine at room temperature (Example 6). Thus. the following advantages of the compounds of general formula (I) as trifluoromethylsulfinylating reagents should be pointed out:
(i) The compounds of the general formula (I) are powerful trifluoromethylsulfinylating agents: alcohols, phenols, amines and heterocyclic compounds can be easily converted into the corresponding trifluoromethylsulfinyl derivative. Electrophilic CF₃S(O)+ group transfer capacity of the compound of general formula (I) is equivalent to or exceeds that of trifluoromethylsulfinyl halides ;
(ii) Many types of trifluoromethylsulfinyl derivatives are easily synthesized by reacting a compound of general formula (I) with a nucleophilic substrate in the absence of any additionnal reagents ;
(iii) In preparating trifluoromethylsulfinyl derivatives starting from low-nucleophilic substrates, the use of polar solvents, organic bases such as triethylamine or high-temperature conditions becomes possible ;
(iv) The only co-product of the reaction : the cyclic imide, is sparingly soluble in organic solvents and can be removed from the product very easily. It is an added advantage that it can be recovered and used again ;
(v) The yields with which the products are obtained are in most cases excellent and the product's purity is also very satisfactory ;
(vi) The above mentioned compounds are solid, non-volatile, stable on storage and much more easily handled than their halide analogues ;
(vii) Both the new reagents and the process of their use meet many economical and environmental requirements.

The following examples illustrate the synthesis of the compounds according to the invention and their use for electrophilic halogenoalkylsulfinylation of organic substrates. It will appreciated that the instant specification is set forth by way of illustration and not limitation, and that various modifications and changes may be made without departing from the spirit and scope of the present invention.

### EXAMPLE 1 :

### N-Trifluoromethylsulfinylimides. General Procedure.

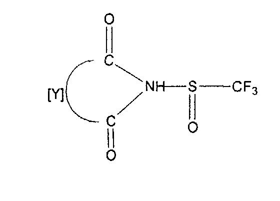

Trifluoromethylsulfinyl chloride (20 mmol) was added dropwise during 5 min under argon to an ice-cooled suspension of N-lithium (sodium, potassium or silver) imide in 30 ml of dry toluene or chlorobenzene. The reaction mixture was stirred at 20 °C for 24 h and filtered. If necessary, additional toluene was added to dissolve the the N-trifluoromethylsulfinylimide and thereby separate it from the insoluble metal chloride. The filtrate was then evaporated in vacuum. By repeated recrystallization from toluene, analytically pure samples were obtained in 65-90 % yield.

### N-Trifluoromethylsulfinylsuccinimide :

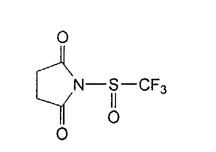

M.p. 76-78 °C. ¹H NMR (C₆D₆): δ1.58 (s); ¹⁹F NMR (C₆D₆): δ 7.0; ¹³C NMR: δ 28.8 (s, CH₂), 124.3 (q, ¹J_{CF} = 340 Hz, CF₃), 172.8 (s, CO).
MS (I/E): m/z = 215 (M⁺); calculated for C₅H₄F₃NO₃S: 215

### N-Trifluoromethylsulfinylphthalimide :

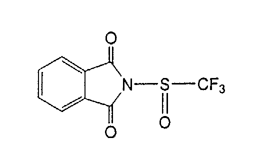

M.p. 126-129 °C. ¹H NMR(C₆D₆): δ □6,8-7,1m); ¹⁹F NMR (C₆D₆): δ 6.5;
MS (I/E): m/z = 263 (M⁺); calculated for C₉H₄F₃NO₃S: 263

### EXAMPLE 2 :

### Trifluoromethylsulfinylation of amines. General procedure.

The amine (10 mmol) was dissolved in 8 ml of toluene and the solution was cooled to O °C. A solution of 10 mmol N-trifluoromethylsulfinylsuccinimide in 5 ml of toluene was added dropwise during 5 min. The reaction mixture was stirred at 20 °C for 30 min and then filtered. Residue after evaporation of solvent on a rotary evaporator can be purified by distillation or flash chromatography. Yield 85-97 %.

### N-Trifluoromethylsulfinyldimethylamine :

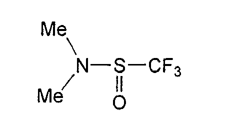

B.p. 42-45 °C (15 mm Hg).
¹H NMR (C₆D₆): δ 2.25 (br. s); ¹⁹F NMR (C₆D₆): δ 0.5; ¹³C NMR: δ 34.6 (s, CH₃), 124.5 (q, ¹J_{CF} = 345 Hz, CF₃).
MS (I/E): m/z = 161; calculated for C₃H₆F₃NOS: 161

### N-Trifluoromethylsulfinyl-N-methylaniline :

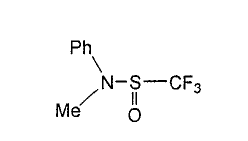

¹H NMR (C₆D₆): δ 3.25 (q, ⁵JHF = 1.5 Hz, CH₃), 7.2-7.4 (m, Hₐᵣₒₘ); ¹⁹F NMR (C₆D₆): δ 2.1.
MS: m/z = 223 (M+). Calculated for C₉H₈F₃NOS: 223.

### EXAMPLE 3 :

### Trifluoromethylsulfinylation of (1S)-endo-(-)-borneol

(a). In the absence of organic base.
   To a solution of borneol (5 mmol) in 4 ml of chlorobenzene a solution of 6 mmol N-trifluoromethylsulfinylsuccinimide in 5 ml of chlorobenzene was added dropwise during 5 min. The reaction mixture was stirred at 80 °C for 3 h and then filtered. Colorless oil after evaporation of solvent in vacuum was purified by flash chromatography. Yield 89 %.
(b). In the presence of triethylamine.
   To a solution of borneol (5 mmol) and N-trifluoromethylsulfinylsuccinimide (5 mmol) in 10 ml chlorobenzene was added at room temperature 5 mmol of triethylamine. The resulting mixture was stirred for 30 min then deposited on chromatography column and eluted with a mixture of pentane and ether (4/1). Colorless oil. Yield 92 %. 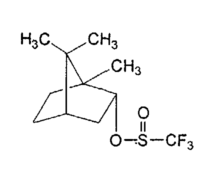
(mixture of diastereoisomer, 50/50)
¹H NMR (C₆D₆): δ 0.59 (s, 6H, CH3), 0.66 (s, 6H, CH₃), 0.69 (s, 3H, CH₃), 0.78 (s, 3H, CH₃), 1.0-2.3 m (8H, CH₂, CH). ¹⁹F NMR (C₆D₆): δ -4.25, -4.90.
MS: m/z = 255 (M-CH₃)+. Calculated for C₁₁H₁₇F₃O₂S: 270.

### EXAMPLE 4 :

### Trifluoromethylsulfinylation of N-methylpyrrole :

N-Methylpyrrole (10 mmol) and N-trifluoromethylsulfinylsuccinimide (12 mmol) were dissolved in 15 ml chlorobenzene and the mixture was heated at 70 °C during 5 h and then poured onto 75 ml of ice-water. The organic phase was separated and dried (Na₂SO₄). ¹⁹F NMR analysis revealed the mixture of 2- and 3-trifluoromethylsulfinylpyrroles (87/13). Flash chromatography with pentane/ether as eluent gives 1-methyl-2-trifluoromethylsulfinylpyrrole as major product. Yellow oil. Yield 67 %. 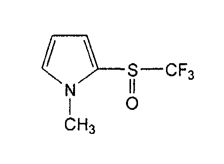 ¹H NMR (C₆D₆): δ 3.64 (s, CH₃), 6.22 (dd, ³J_{HH} = 4.1 Hz, ³J_{HH} = 2.6 Hz, H⁴), 6.81 (dd, ³J_{HH} = 4.1 Hz, ⁴J_{HH} = 1.7 Hz, H³), 6.90 (dd, ⁴J_{HH} =1.7 Hz, ³J_{HH} = 2.6 Hz, H⁵).
¹⁹F NMR (C₆D₆): δ 2.9.
MS: m/z = 197. Calculated for C₆H₆F₃NOS: 197.

### EXAMPLE 5 :

### Trifluoromethylsulfinylation of 1-phenyl-3-methyl-5-aminopyrazole :

1-Phenyl-3-methyl-5-aminopyrazole (10 mmol) and N-trifluoromethylsulfinyl-succinimide (12 mmol) were dissolved in 15 ml chlorobenzene and the mixture was heated at 70 °C during 3 h and then poured onto 75 ml of ice-water. The organic phase was separated and dried (Na₂SO₄). Flash chromatography with chloroform as eluent gives 1-Phenyl-3-methyl-4-trifluoromethylsulfinyl-5-aminopyrazole. Yield 82 %. 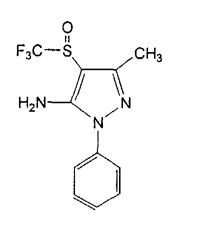 M.p. 90-93 °C.
¹H NMR (C₆D₆): d 1.97 (s, 3H, CH₃), 4.53 (s, 2H, NH₂), 6.88-7.23 (m, 5H, C₆H₅). ¹⁹F NMR (C₆D₆): d -0.38 (CF₃)).
MS: m/z 289 (M+), calculated for C₁₁H₁₀F₃N₃OS: 289

### EXAMPLE 6 :

### Trifluoromethylsulfinylation of 1-(2,6-dichloro-4-trifluoromethylphenyl)-3-cyano-5-aminopyrazole

To a solution of 3 mmol of 1-(2,6-dichloro-4-trifluoromethylphenyl)-3-cyano-5-aminopyrazole in 15 ml dichloromethane was added 3.1 mmol triethylamine. This mixture was stirred for 15 min. A solution of N-trifluoromethylsulfinylsuccinimide (3.1 mmol) in 7 ml dichloromethane was then added during 10 min at 10 °C . After stirring 3 h at room temperature ¹⁹F NMR analysis revealed the quantitative formation of N-trifluoromethylsulfinylaminopyrazole. Succinimide and triethylamine were removed by fast extraction with ice-water. The organic phase was dried (Na₂SO₄) and evaporated. The crude N-Trifluoromethylsulfinylaminopyrazole can be without further purification transformed via "Thia-Fries Rearrangement" into 1-(2,6-dichloro-4-trifluoromethylphenyl)-3-cyano-4-trifluoromethylsulfinyl-5-aminopyrazole in conditions known for rearrangement of sulfinylanilides into sulfoxides, for example, at 35-55 °C in the presence of 1 equivalent of hydrochloric acid (see, K.K. Andersen and O. Malver, J. Org. Chem., 1983, vol. 48, p. 4803; O. Cameron et al., J.Org. Chem., 1980, vol. 45, p. 5336). Yield 70% 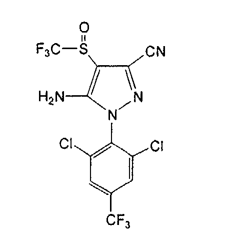 M.p. 206-208 °C. ¹H NMR (CDCl₃): δ 5.15 (s, 2H, NH2), 7.83 (s, 2H, Hₐᵣₒₘ). ¹⁹F NMR (CDCl₃): d 0.21 (s, CF₃SO), 11.73 (s, CF₃-*Ar*).

In the transformation above-mentionned , it can be pointed out that the crude N-trifluoromethylsulfinylaminopyrazole can be isolated and purified . The purification of the residue after dichloromethane evaporation by crystallization from toluene gives 1-(2,6-dichloro-4-trifluoromethylphenyl)-3-cyano-5-trifluoromethylsulfinylaminopyrazole 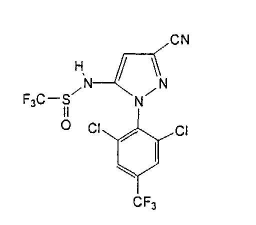 M.p. 125-128 °
¹H NMR (d₆-acetone): δ 7.0 (s, 1H), 8.1 (s, 2H). ¹⁹ F NMR (C₆D₆): δ - 2.5;
MS (IE): m/z 436 (M⁺), calculated for C₁₂H₄Cl₂F₆N₄OS: 436.

In a similar manner , the following compounds hereafter known as examples 7 to 12 were prepared :

### EXAMPLE 7 :

-From N trifluoromethylsulfinylsuccinimide and 1-( 2,6-dichloro-4-trifluoromethylphenyl) -3-trifluoromethyl -5-aminopyrazole , we obtained 1-(2,6-dichloro-4-trifluoromethylphenyl)-3-trifluoromethyl-4-trifluoromethylsulfinyl-5-aminopyrazole.
M.p.:107-112°C ; 1H NMR ( d6 acetone) : 6.76 ( s, 2H , NH2) , 8,13 ( s , 2H , H arom ). 19F NMR ( d6 acetone ): 0.18 ( s, CF3SO ), 12,33 ( s , CF3 arom ) , 13,79 ( s, 3-CF3 )

### EXAMPLE 8 :

From N-trifluoromethylsulfinylsuccinimide and 1-(2,4,6-trichlorophenyl)-3-cyano-5-aminopyrazole , we obtained 1-(2,4,6-trichlorophenyl)-3-cyano-4-trifluoromethylsulfinyl-5-aminopyrazole
M.p.: 186-189°C ; 1H NMR ( CDCl3 ) : 5,20 ( s , 2H , NH2 ), 7,50 ( s, 2H arom )

### EXAMPLE 9 :

From N-trifluoromethylsulfinylsuccinimide and 1-( 2,6-dichloro-4-trifluoromethylphenyl)-3-carboxamido-5-aminopyrazole, we obtained 1-(2,6-dichloro-4-trifluoromethylphenyl)-3-carboxamido-4-trifluoromethylsulfinyl-5-aminopyrazole
M.p.: 231-233°C ; 1-H NMR ( d6 acetone ) : 6.39 (s, 2H , NH2 ), 6.87 ( s , 1H, CONH2 ), 7.25 ( s , 1H, CONH2 ), 8.11 ( s, 2H, H arom ) ; 19F NMR ( d6 acetone ): 0.10 ( s, CF3SO ), 12.43 ( s, CF3 arom )

### EXAMPLE 10 :

From N-trifluoromethylsulfinylsuccimide and 1-(2-bromo-4-trifluoromethyl-6-chlorophenyl)-5-aminopyrazole we obtained 1-(2-bromo -4-trifluoromethyl-6-chlorophenyl)-4-trifluoromethylsulfinyl-5-aminopyrazole
M. p. : 82-86°C

### EXAMPLE 11:

From N-trifluoromethylsulfinylphtalimide and 1-(2,6-dichloro-4-trifluoromethoxyphenyl)-5-aminopyrazole , we obtained 1-(2,6-dichloro-4trifluoromethoxyphenyl)-4-trifluoromethylsulfinyl-5-aminopyrazole
M p. : 55-57°C

### EXAMPLE 12 :

From N-trifluoromethylsulfinylphtalimide and 1-(2-chloro-4-trifluoromethylphenyl)-5-aminopyrazole , we obtained 1-(2-chloro-4-trifluoromethylphenyl)-4-trifluoromethylsulfinyl-5-aminopyrazole
M.p. : 132-134°C

## Claims

1. A compound corresponding to formula (I) : 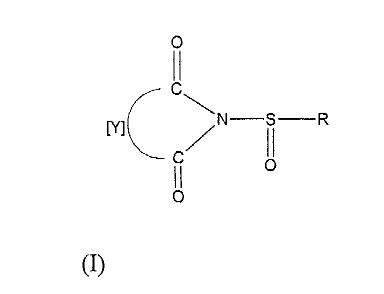 wherein :
- Y represents a group chosen from : a C₁-C₂₀, linear, branched or cyclic alkane di-yl, or alkene di-yl, or alkyne di-yl radical, wherein said alkane, alkene or alkyne chain can be substituted by one or more groups chosen from : a phenyl ring or a halogen atom, and said alkane, alkene or alkyne chain can comprise a sulfur bridge or an oxygen bridge ;
- R represents an alkyl group, linear or branched, comprising one to four carbon atoms and substituted by one or more, identical or different, halogen atoms.

2. Compound of general formula (I) according to claim 1, wherein Y is chosen so that the radical of formula (III): 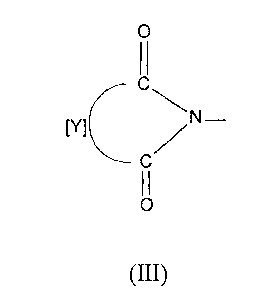 is a 5- or 6-membered cyclic dicarboximide.

3. Compound of general formula (I) according to claim 1 or claim 2, wherein Y is chosen so that the radical of formula (III) : 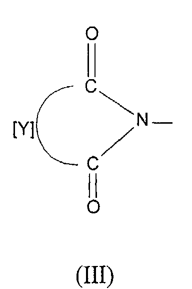 is one of the following group : succinimidyl (A), tetrahydrophthalimidyl (B), endo-5-norbornene-3,3-dicarboximidyl (C), 2,4-thiazolidenedion-3-yl (D), glutarimidyl (E), maleinimidyl (F), phtalimidyl (G), tetrachlorophthalimidyl (H), 1,8-naphthalimidyl (I), pyrromellitic diimidyl (J):

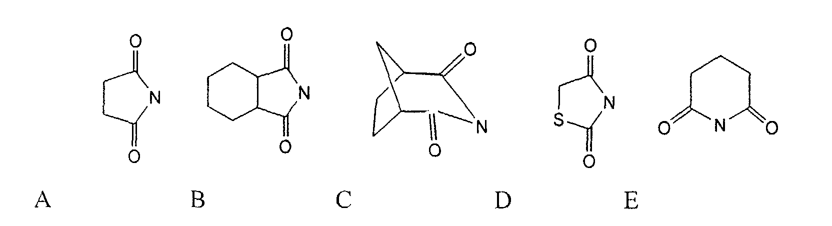

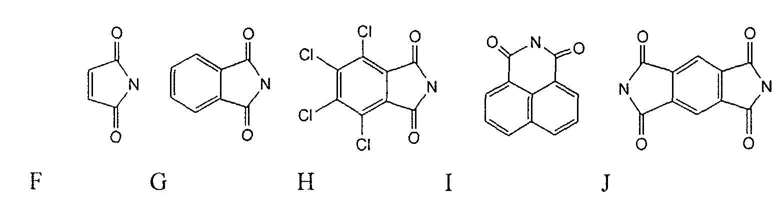

4. Compound of general formula (I) according to any one of claim 1 to claim 3, wherein Y is chosen so that the radical of formula (III) : 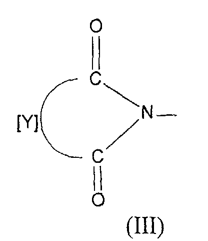 represents a group chosen from: succinimidyl (A), phthalimidyl (G) and tetrachlorophthalimidyl (H).

5. Compound of general formula (I) according to any one of claim 1 to claim 4, wherein R represents trifluoromethyl.

6. Process for the preparation of a compound according to any one of claim 1 to 5, **characterised in that** the corresponding N-metal substituted cyclic imide and halogenoalkylsulfinylchloride are reacted together as represented in Reaction 1 : 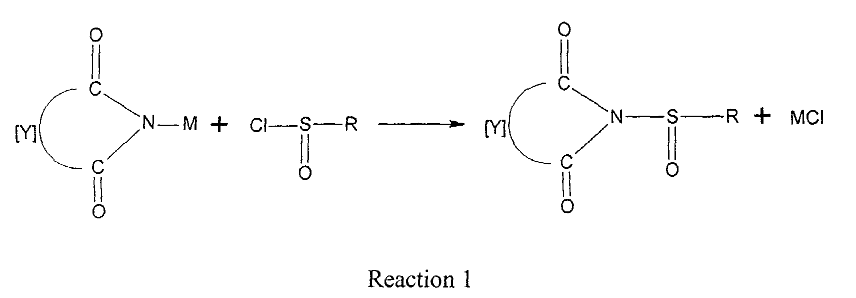 wherein M represents a metal atom and Y and R have the same meaning as in claim 1 to 5.

7. Process according to claim 6, **characterised in that** R is trifluoromethyl.

8. Process according to claim 6 or claim 7 **characterised in that** M represents a lithium, sodium, potassium, magnesium, copper or silver atom.

9. Process according to any one of claim 6 to 8, **characterized in that** the reaction is made in a halogenocarbonated solvent.

10. Method for the introduction of a halogenoalkylsulfinyl group into an organic substrate noted NuH **characterised in that** a compound of general formula (I) according to any one of claim 1 to 5, wherein Y and R have the same signification as in claim 1 to 5, is reacted with NuH (Reaction 2):

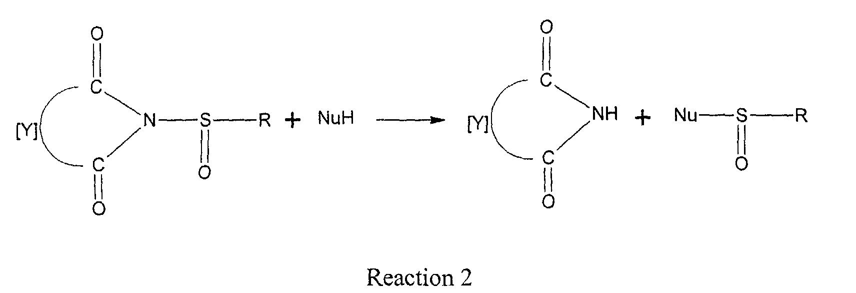

11. Method according to claim 10, **characterised in that** 1.0 to 1.2 moles of a compound of the general formula (I) are employed per mole of nucleophilic substrate.

12. Method according to claim 10 or claim 11, **characterised in that** the nucleophilic substrate is chosen from : alcohols, phenols, amines and heterocyclic compounds.

13. Method according to any one of claim 10 to claim 12, **characterised in that** the nucleophilic substrate is chosen from : pyrroles, pyrazoles, imidazoles, oxazoles, isoxazoles, isothiazoles, thiazoles, triazoles, said heterocycles being possibly substituted by one or more atom or groups chosen from : a halogen atom, -NH₂, a C₁-C₁₀ mono- or di-alkylamine, a nitrile, one or more C₁-C₁₀ alkyl or C₁-C₁₀ halogenoalkyl group, an aryle group, said aryle group being possibly substituted by one or more halogen atom, one or more C₁-C₁₀ alkyl or C₁-C₁₀ halogenoalkyl group.

14. Method according to any one of claim 10 to claim 13, **characterised in that** the reaction is made in a solvent chosen from the group consisting of chlorinated hydrocarbons and aromatic, optionally halogenated hydrocarbons.

15. Method according to any one of claim 10 to claim 14, **characterised in that** the solution of compound (I) in an organic solvent is added at temperature between 0 °C and 90 °C to the nucleophilic substrate NuH.

16. Method according to any one of claim 10 to claim 15, **characterised in that** in the reaction is done in the presence of an organic base.

17. Method according to any one of claim 10 to claim 16, **characterised in that** in the cyclic imide resulting from reaction 2 is regenerated and reconverted into compound (I).

18. Method according to any one of claim 10 to claim 17, **characterised in that** R is CF₃.

19. Method according to any one of claim 10 to claim 18, **characterised in that** compound (I) is chosen from the group consisting of: N-trifluoromethylsulfinylsuccinimide and N-trifluoromethylsulfinylphthalimide.

20. Method according to any one of claim 10 to claim 19, **characterised in that** Nu is chosen from the group consisting of: dimethylamine, N-methylaniline, (1S)-endo-(-)-borneol, N-methylpyrrole, 1-phenyl-3-methyl-5-aminopyrazole, 1-( 2,6-dichloro-4-trifluoromethylphenyl) -3-trifluoromethyl -5-aminopyrazole, 1-(2,4,6-trichlorophenyl)-3-cyano-5-aminopyrazole, 1-( 2,6-dichloro-4-trifluoromethylphenyl)-3-carboxamido-5-aminopyrazole , 1-(2-bromo-4-trifluoromethyl-6-chlorophenyl)-5-aminopyrazole, 1-(2,6-dichloro-4-trifluoromethoxyphenyl)-5-aminopyrazole, 1-(2-chloro-4-trifluoromethylphenyl)-5-aminopyrazole, 1-(2,6-dichloro-4-trifluoromethylphenyl)-3-cyano-5-aminopyrazole.
